(19) Europäisches Patentamt
European Patent Office
Office européen des brevets

(11) Publication number: **0 407 190 A2**

# (12) EUROPEAN PATENT APPLICATION

(21) Application number: **90307347.6**

(51) Int. Cl.[5]: **C12P 41/00**, C12P 13/04

(22) Date of filing: **05.07.90**

(30) Priority: **07.07.89 US 376448**

(43) Date of publication of application:
**09.01.91 Bulletin 91/02**

(84) Designated Contracting States:
**GR**

(71) Applicant: **SCHERING CORPORATION**
**2000 Galloping Hill Road**
**Kenilworth New Jersey 07033(US)**

(72) Inventor: **Schumacher, Doris P.**
**76 Edgewood Drive**
**Florham Park, New Jersey 07932(US)**
Inventor: **Clark, Jon E.**
**501 Grove Avenue**
**Highland Park, New Jersey 08904(US)**
Inventor: **Walker, Derek**
**38 Gloucester Road**
**Summit, New Jersey 07901(US)**

(74) Representative: **Ritter, Stephen David et al**
**Mathys & Squire 10 Fleet Streeteet**
**London EC4Y 1AY(GB)**

## (54) Process for producing antibacterial intermediates via enzyme hydrolysis of racemic substrates.

(57) A novel process for preparing antibacterial intermediates, such as for preparing the antibacterial compound thiamphenicol, is disclosed which utilizes a γ-glutamyl hydrolase to selectively hydrolyze a racemic mixture of D-Threo and L-threo phenylserines.

EP 0 407 190 A2

# PROCESS FOR PRODUCING ANTIBACTERIAL INTERMEDIATES VIA ENZYME HYDROLYSIS OF RACEMIC SUBSTRATES

## BACKGROUND OF THE INVENTION

Thiamphenicol is a valuable and effective antibacterial in worldwide use. The current manufacturing process involves a classical resolution of D,L-threo-2-amino-1-(4-methyl-mercaptophenyl)-1,3-propanediol. The D isomer is converted to thiamphenicol and the L isomer is accumulated as waste. One solution to this problem would be to resolve an earlier intermediate in the thiamphenicol process, i.e. an N-acyl or N-aroyl derivative of 4-methylmercaptophenylserine. The unwanted L-isomer could then be converted to the desired D-isomer as described in the literature (Japan Kokai 75,148,326 27 November 1975, Appl. 74 49 370,01 May, 1974). Although enzymatic hydrolysis of amides is a known process for the resolution of amino acids ("Chemistry of the Amino Acids, Volume 1, Jesse P. Greenstein and Milton Winitz, John Wiley and Sons, 1954, pp 728-743), it has not been used for the resolution of substituted phenylserines. We have discovered that a certain amidase, ie. an enzyme that will convert amides to amines, can selectively hydrolyze the amide bond of one enantiomer in a racemic mixture and thus allow the desired separation of D,L-threo amides.

## SUMMARY OF THE INVENTION

In its first embodiment, the present invention is directed toward a process for separating enantiomer amides in a racemic mixture of the formula:

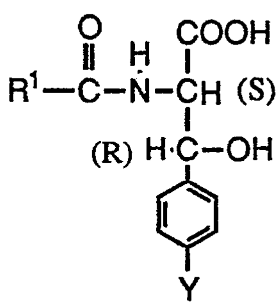

D-Threo
(S,R)

and

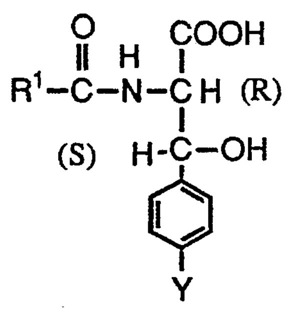

L-Threo
(R,S)

(I) (II)

wherein $R^1$ is alkyl, substituted alkyl, alkenyl, substituted alkenyl, aryl, substituted aryl, aralkyl, substituted aralkyl, aromatic heterocyclic, substituted aromatic heterocyclic, heterocyclic alkyl or substituted heterocyclic alkyl and Y is -H, $-NO_2$, $-S(O)xCH_3$ wherein

x is 0, 1, 2; comprising

(a) selectively hydrolyzing one of the enantiomer amides in the racemic mixture with an enzyme to its corresponding amine or salt having an enantiomeric purity of about 80% percent or greater while leaving the other enantiomer amide substantially unhydrolyzed; and

(b) separating the amine or salt from the unhydrolyzed amide.

In a second embodiment, the present invention further comprises step

(c) converting both the separated amine or salt and the substantially unhydrolyzed amide into a compound of the formula

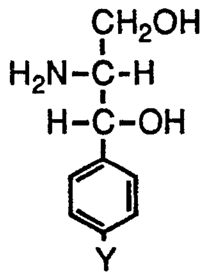

D-Threo (R,R)

(III)

wherein Y is defined as hereinbefore.

## DETAILED DESCRIPTION OF THE INVENTION

As used herein, unless otherwise stated, the term "alkyl" refers to a straight chain saturated hydrocarbon moiety containing from 1 to 6 carbon atoms, or a branched saturated hydrocarbon moiety of 3 to 6 carbon atoms, such as for example, methyl (i.e. $-CH_3$), ethyl, propyl, isopropyl, butyl, isobutyl, tert-butyl, pentyl, hexyl and the like; the term "substituted alkyl" refer to an alkyl moiety which is further substituted at the carbon by one or more of the following groups: halo, alkyl of one to six carbon atoms, aryl of six to fifteen carbon atoms, cyano (i.e. -CN), carboxyl (i.e. -COOH) or salts thereof, nitro (i.e. $-NO_2$) and hydroxyl;
the term "alkenyl" refers to a straight chain hydrocarbon moiety of two to six carbon atoms or a branched hydrocarbon moiety of three to six carbon atoms having at least one carbon to carbon double bond such as allyl, ethenyl, propenyl, 1-butenyl, 2-butenyl, isobutenyl, 1-pentenyl, 2-methyl-1-butenyl, 1-hexenyl and the like; the term "substituted alkenyl" refer to an alkenyl moiety which is further substituted at the carbon by one or more of the following groups: halo, alkyl of one to six carbon atoms, aryl of six to fifteen carbon atoms, cyano (i.e. -CN), carboxyl (i.e. -COOH) or salts thereof, nitro (i.e. $-NO_2$) and hydroxyl;
the term "aryl" refers to a carbocyclic moiety containing at least one benzenoid-type ring, with the aryl groups preferably containing from 6 to 15 carbon atoms, for example, phenyl, naphthyl, indenyl, indanyl and the like; the term "substituted aryl" refer to an aryl moiety which is further substituted at the carbon by one or more of the following groups: halo, alkyl of one to six carbon atoms, aryl of six to fifteen carbon atoms, cyano (i.e. -CN), carboxyl (i.e. -COOH) or salts thereof, nitro (i.e. $-NO_2$) and hydroxyl;
the term "aralkyl" refers to an aryl moiety of six to 15 carbon atoms covalently bonded to an alkyl moiety of one to six carbon atoms, for example, benzyl, phenylethyl, and the like; the term "substituted aralkyl" refer to an aralkyl moiety which is further substituted at the carbon by one or more of the following groups: halo, alkyl of one to six carbon atoms, aryl of six to fifteen carbon atoms, cyano (i.e. -CN), carboxyl (i.e. -COOH) or salts thereof, nitro (i.e. $-NO_2$) and hydroxyl;
the term "aromatic heterocyclic" refers to a cyclic moiety having at least one oxygen (O), sulfur (S) and/or nitrogen (N) heteroatom interrupting the ring structure and having a sufficient number of unsaturated carbon to carbon bonds, nitrogen to carbon bonds, and the like, to provide aromatic character, with the aromatic heterocyclic groups preferably containing from 2 to 14 carbon atoms, for example, 2-, 3- or 4-pyridyl, 2- or 3-furyl, 2- or 3-thienyl, 2-, 4- or 5-thioazolyl, 2-, 4-or 5-imidazolyl, 2-, 4- or 5-pyrimidinyl, 2-pyrazinyl, 3- or 4-pyridazinyl, 3-, 5- or 6[1,2,4-triazinyl], 3- or 5-[1,2,4-thiadiazolyl], 2-, 3-, 4-, 5-, 6- or 7-benzofuranyl, 2-,3-, 4-, 5-, 6- or 7-indolyl, 3-, 4- or 5-pyrazolyl, 2-, 4- or 5-oxazolyl, and the like; the term "substituted aromatic heterocyclic" refer to an aromatic heterocyclic moiety which is further substituted at a carbon or heteroatom by one or more of the following groups: halo, alkyl of one to six carbon atoms, aryl of six to fifteen carbon atoms, cyano (i.e. -CN), carboxyl (i.e. -COOH) or salts thereof, nitro (i.e. $-NO_2$) and hydroxyl;
the term "heterocyclic alkyl" refers to an aromatic heterocyclic moiety of 2 to 14 carbon atoms as defined hereinbefore, covalently bonded to an alkyl moiety of one to six carbon atoms; the term "substituted heterocyclic alkyl" refer to a heterocyclic alkyl moiety which is further substituted at a carbon or heteroatom by one or more of the following groups: halo, alkyl of one to six carbon atoms, aryl of six to fifteen carbon atoms, cyano (i.e. -CN), carboxyl (i.e. -COOH) or salts thereof, nitro (i.e. $-NO_2$) and hydroxyl;
the terms "halogen" and "halo" refer to fluorine, chlorine, bromine or iodine;
the term "enantiomeric purity" refers to the amount of one enantiomer of either the hydrolyzed amine (IV) or the unhydrolyzed amide (II). As described in The Vocabulary of Organic Chemistry, Milton Orchin, Fred

Kaplan, Roger S. Macomber, R. Marshall Wilson and Hans Zimmer, Wiley-Interscience John Wiley and Sons, New York, 1980, pp 130, an enantiomeric purity of 100 percent means only one hydrolyzed enantiomer is present. Likewise, an enantiomeric purity of 50 percent indicates that both enantiomers in a racemic mixture are equally hydrolyzed, i.e. there is no selectivity.

The term "enzyme" is intended to mean in its broadest sense, an organic catalyst produced by living cells but capable upon separation from the cell or upon chemical modification, of acting independently. The term "amidase", a specific type of enzyme, is as defined in George M. Whitesides and Chi-Huey Wong, Angewandte Chemie, Int. Ed., Volume 24, 1985, pp 617-718. The amidase employed in the present invention is a $\gamma$-glutamyl hydrolase, such as Enzyme Commission (EC) 3.4.22.12. The isolation and description of this enzyme is described in Levy, C.C. and Goldman, P., Biol. Chem. 242, 2933 (1967). The enzyme is also commercially available from the Signa Chemical Co., P.O. Box 14508, St. Louis, Missouri, U.S.A.

The procedure for preparing these compounds can be represented as follows:

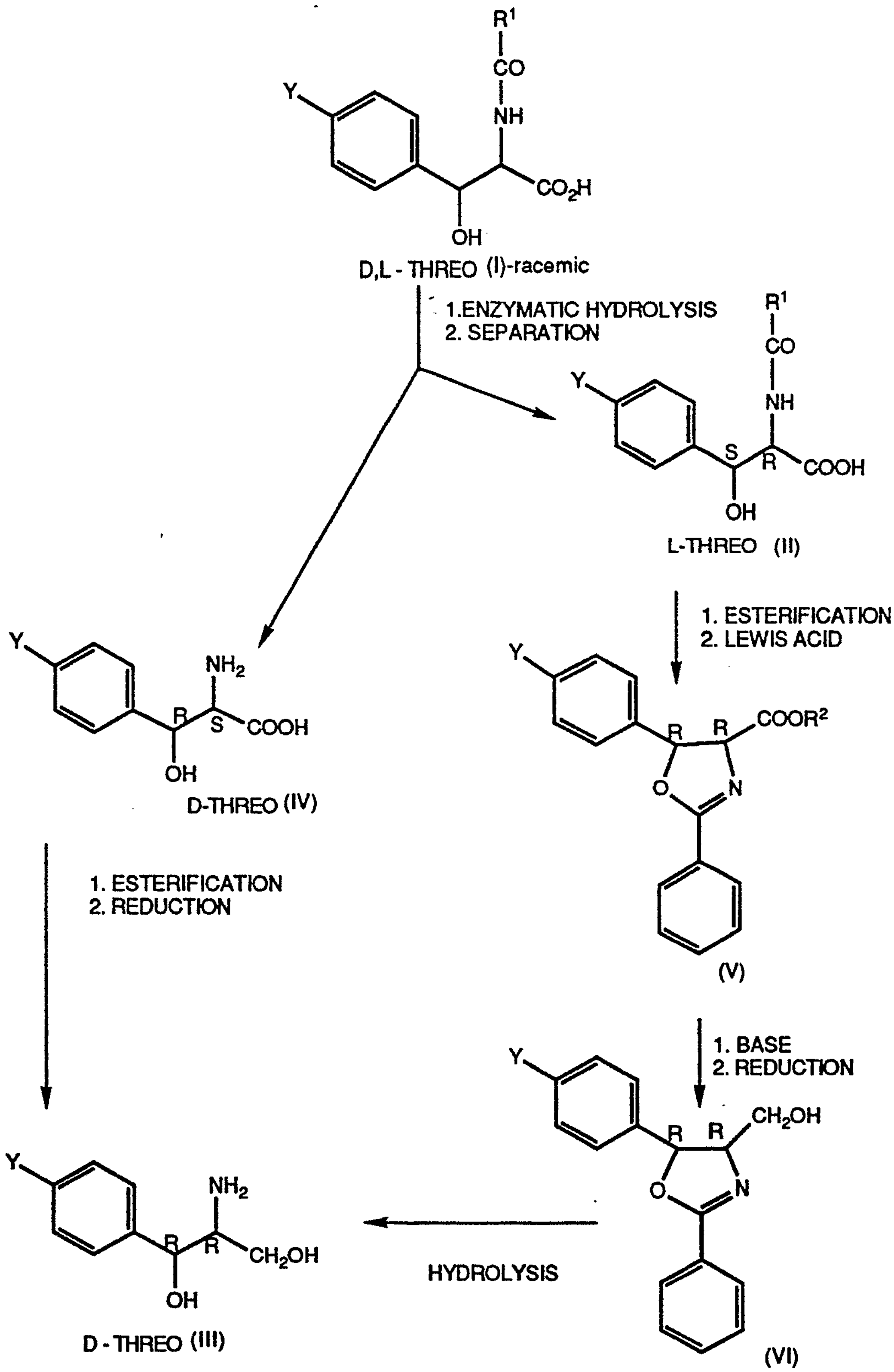


D,L - THREO (I)-racemic

L-THREO (II)

D-THREO (IV)

(V)

(VI)

D - THREO (III)

In step (a), enantiomer amides I and II in the racemic mixture are distinguished by selectively hydrolyzing one of the enantiomer amides, i.e. I or II to its corresponding amine or salt with an enzyme, and leaving the other enantiomer substantially unhydrolyzed. The amine or salt is then separated by acid extraction or other conventional means for separation such as chromatography. The particular amide which is selectively hydrolyzed should be hydrolyzed to an amine or salt having an enantiomeric purity of about 80 percent (%) or greater. More preferably, the enzyme should be able to selectively hydrolyze one of the

enantiomer amides to its corresponding amine or salt in an enantiomeric purity of about 90 percent or greater, even more preferably about 94 percent or greater, most preferably about 97 percent or greater. The enzyme can be in free or immobilized form, preferably free. Methods for immobilizing such enzymes can be found in Methods of Enzymology, Volume XLIV, Ed. by Klaus Mosbach, Academic Press, New York, 1976.

The racemic mixture is contacted with a suitable enzyme in amounts and under conditions effective to selectively hydrolyze one of the enantiomer amides. In a batch procedure the amount of enzyme used in the present method can range from about 1 g to about 1 µg of enzyme per one gram of enantiomer amides I and II, preferably from about 0.01 g to about 0.5 g of enzyme per gram of enantiomer amide. However, where an immobilized enzyme is employed, the amount of enzyme can be less than the amounts described above. The contacting can be carried out in an aqueous medium, preferably distilled or deionized water, or water mixed with a suitable miscible or immiscible organic solvent. Suitable organic solvents include but are not limited to $C_1$ to $C_6$ alcohols, such as methanol and ethanol, dihydric or trihydric alcohols such as glycol or glycerol, or other solvents such as dimethylsulfoxide, acetonitrile, dimethylformamide, dimethylacetamide, acetone, methylethyl ketone and tetrahydrofuran, toluene, hexane, tert-butyl methyl ether, methylethyl ketone, methyl isobutyl ketone, methylene chloride, or mixtures of any of the above. The organic solvents can be mixed with water in concentrations up to 50% by volume or more but preferably up to about 10% by volume.

Optionally, the aqueous medium can contain a suitable buffer, such as those described in Gordon and Ford (Ed.). The Chemist's Companion: A Handbook of Practical Data, Techniques and References, pp. 71 (1972), whose preparative teachings are incorporated herein by reference. The aqueous medium is maintained between a pH of about 4 to 9, preferably a pH of about 5.5 to 8, more preferably a pH of about 5.5 to 7. Any buffering agent used can be employed in amounts ranging from about zero to about one mole/liter (M), preferably about 0.05M. Alternatively, a suitable base can be added to neutralize the acid produced during hydrolysis. Suitable bases include sodium and potassium hydroxides, carbonates, bicarbonates and mixtures thereof.

The selective hydrolysis of one of the enantiomer amides in step (a) can be carried out at temperatures ranging from about -5 degrees Celsius (°C) to about 80°C, preferably from about 10 to about 45°C, more preferably from about 30 to about 35°C. The reactants are preferably stirred for a time effective to selectively hydrolyze one of the enantiomer amides to its corresponding amine or salt, ranging from about 30 minutes to about 48 hours or more until the reaction is completed to the extent desired.

In step (b) the substantially unhydrolyzed enantiomer amide (II) can be separated ie. recovered from the reaction mixture, by extracting the aqueous layer with a suitable water-immiscible solvent at a pH of about 6 or lower. Such water-immiscible solvents include but are not limited to the chlorinated hydrocarbons such as chlorform, carbon tetrachloride, and methylene chloride, the alkyl esters such as ethyl acetate, isopropyl acetate and ethyl benzoate, the aromatic hydrocarbons such as toluene and xylenes, and ethers such as tert-butylmethyl ether (tert-BuOMe). Following extraction, the water-immiscible solvent can be removed by conventional procedures such as distillation, and the like to give the unhydrolyzed enantiomer amide.

The remaining acidic aqueous layer can be made basic, filtered and concentrated by conventional procedures such as lyophilization, distillation and the like to give the desired amine or salt. Alternatively, the amine or salt may be separated from the unhydrolyzed ester employing chromatographic procedures such as described in Snyder and Kirkland, Introduction to Modern Liquid Chromatography, 2nd ed., Wiley-Interscience, New York, 1979. For example, after selective hydrolysis of the enantiomer amide, the reaction mixture can be chromatographed over gravity based or normal phase silica gel columns using suitable eluting solvents.

The separated amine or salt and the substantially unhydrolyzed enantiomer amide can be converted to desired compounds III by the following procedures.

After the amine or salt is separated from the unhydrolyzed enantiomer amide, the carboxylic acid can be esterified to any ester desired using conventional esterification procedures, such as described in Jerry March, Advanced Organic Chemistry, Reactions, Mechanisms and Structure, John Wiley and Sons, New York, New York, 1985, 1346 pp., whose preparative teachings are incorporated herein by reference.

The D-threo amine (S,R) of formula IV can be converted to the D-Threo alcohol (R,R) of formula III by esterification followed by reduction of the ester moiety using known reducing agents and under conditions such as described in March, supra. Suitable reducing agents include $LiAlH_4$, $LiBH_4$, $Ca(BH_4)_2$ prepared by $CaCl_2$ and $NaBH_4$. Alternatively, the ester can be reduced with a hydrogenating agent, including the requisite hydrogenating catalyst(s) and hydrogen. Various selected catalysts and conditions are described in "Catalytic Hydrogenation in Organic Synthesis", (1978) Morris Freifelder, Chapter 4, Olefins, pages 15-25, John Wiley and Sons and in March, supra. The hydrogenating catalyst can be nickel, palladium,

platinum, platinum oxide, platinum on carbon, and mixtures thereof. The source of hydrogen can be hydrogen gas or isotopic forms thereof, such as deuterium or tritium.

The L-threo amide (R,S) of formula II can be converted to the D-threo amine of formula III, by procedures similar to those described in Japan Kokai 75,148,326 27 November 1975, Appl. 74 49 379,01 Nat 1974. Essentially, the L-threo amide (R,S) of formula II is esterified to give the L-threoamide ester (R,S). The L-threoamide (R,S) is treated with a Lewis acid to form a leaving group with the benzylic carbinol and to induce cyclization with inversion of the stereoisomeric center, forming the oxazoline (R,R) of formula V. Suitable Lewis acids for the mediated cyclization with inversion of L-threo (R,S) include but are not limited to thionyl chloride ($SOCl_2$), phosphorus trichloride, phosphorus oxychloride, boron trichloride and phosphorus pentachloride, preferably thionyl chloride.

The oxazoline (R,R) of formula V thus prepared can be treated with a base effective to cause base epimerization of the oxazoline (R,R) ester to the oxazoline (S,R). The base should be strong enough to abstract the proton from the carbon atom connected to the nitrogen atom. Suitable bases include sodium methylate, sodium ethylate, Hunig's base, tetramethylguanidine or triethylenediamine, commercially available as DABCO®, trademark of Air Products and Chemicals, Inc. Allentown, Pennsylvania. Preferably, the base is sodium ethylate.

The oxazoline ester can be converted to the corresponding oxazoline carbinol (R,R) of formula VI according to the same reduction procedures as described above for converting the D-Threo (S,R) ester to the (R,R) D-Threo alcohol.

The oxazoline (R,R) of Formula VI can be fully hydrolyzed with any suitable acid to give the D-threo aminodiol (R,R) of Formula III.

It can thus be appreciated that compound III, prepared from both the separated D-threo (S,R) and L-threo (R,S) compounds can be combined to efficiently utilize each enantiomer starting material in the racemic mixture.

## EXAMPLE

## HYDROLYTIC RESOLUTION OF D. L-THREO-N-BENZOYL-4-METHYLTHLOPHENLSERINE

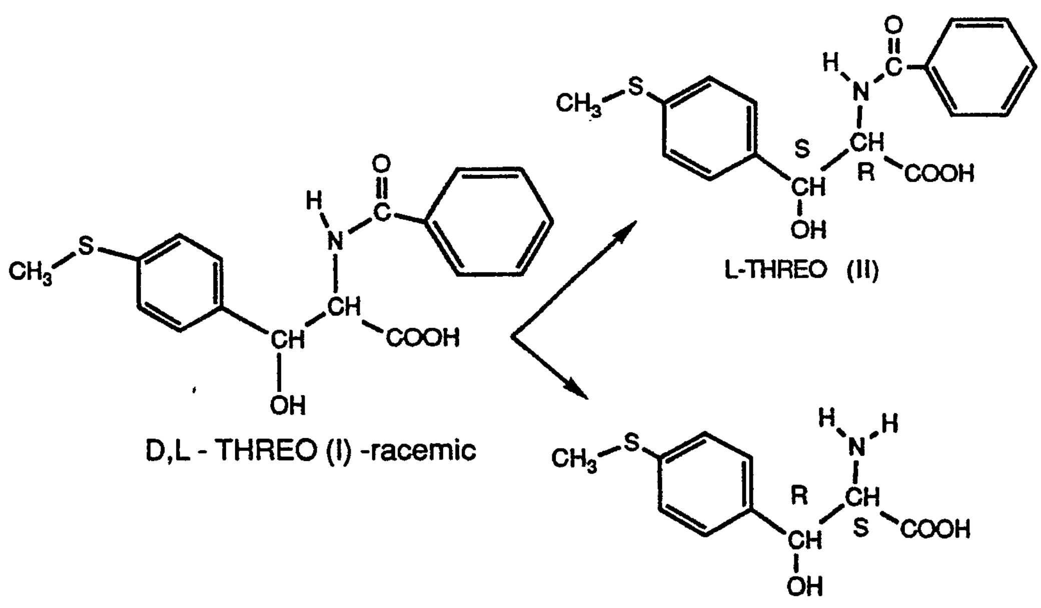

L-THREO (II)

D,L - THREO (I) -racemic

D-THREO (IV)

One hundred milligrams (mg) D,L-threo-N-Benzoyl-4-methylthiophenylserine (I) (molecular weight 332.3, 0.3 millimoles (mmole)) is dissolved in 150 milliliters (ml) pH 7.6 buffer. The buffer is prepared by mixing

1.183 grams (g) potassium phosphate ($KH_2PO_4$) and 5.29 g dipotassium phosphate ($K_2HPO_4$)/liter water. Gamma ($\gamma$)-glutamyl hydrolase is added as follows: 40 mg immediately, 24 mg after 7 days, and 6 mg after 18 days. The reaction mixture is assayed after 6, 12, 18 and 36 days with the chromatographic system: Column Zorbax®C-8 (trademark of Dupont Co., Wilmington, Delaware) , Solvent 1/1 $H_2O$/MeOH 0.5 percent (%) trifluoroacetic acid (TFA), and 1 ml/minute at 254 nanometer (nm). After 36 days the reaction is completed and the reaction mixture is acidifed with hydrochloric acid (HCl) to pH 3 and extracted twice with 250 ml methylene chloride. Sodium chloride is added to break the emulsion. The combined organic layers are back extracted with 50 ml pH 3 buffer. The buffer is prepared as follows: 6.5 g $KH_2PO_4$/liter $H_2O$ acidified to pH 3.0 with phosphoric acid ($H_3PO_4$). The aqueous layers are combined and adjusted to pH 7 with saturated sodium bicarbonate solution. The unhydrolyzed amide (II) is isolated by drying the organic layer with sodium sulfate and concentrated to give 34 mg L-threo(II), a 65% yield. High performance liquid chromatography (HPLC) analysis shows the amide (II) to have an enantiomeric purity of 97%. Chromatographic System Column: YMC C-4 (YMC Co. Ltd., 566 Toraya-Cho Sagaru Oike Karasuma Nakagyoku, Kyoto, Japan), 4.6x25cm, 300 Å, 5u, Solvent: 96% (20 g $\beta$-cyclodextrin, 6.5 g $KH_2PO_4$/l$H_2O$), 4% Acetonitrile and 2 ml/min at 254 nm. The aqueous layer containing amine (IV) is concentrated to give 13.69 g of an off white solid, made of various phosphate salts, sodium chloride and the amine(IV), a 54% yield. This is anaylzed by chromatographic system column: Crownpak CR (+) Solvent (Daicel Chemical Industries, Ltd., New York, New York): 85% water, 15% MeOH 1ml/min acidified to pH 1.5 with $HClO_4$ at 254 nm which shows an 88% enantiomeric purity (ee) of the amine (IV).

PREPARATION OF STARTING MATERIALS

The D,L-threo compound, a mixture of formulas I and II , is known to those skilled in the art, as taught in Japan Kokai 75,148,326 27 November 1975, (Appl. 74 49 370,01 May 1974) and C. Giordano, S. Cavicchioli, S. Levi and M. Villa, New Strategy for Racimization of 2-amino-1,3-propanediols, Key Intermediate for the Synthesis of Antibiotic drugs, Tetrahedron Letters, Vol. 29, No. 43, pp. 5561-5564. The amides of such compounds can be prepared by known procedures, such as those described in Jerry March, Advanced Organic Chemistry, Reactions, Mechanisms and Structures, John Wiley and Sons, New York, New York, 1985, 1346 pp, whose preparative teachings are incorporated herein by reference.

**Claims**

1. A process for separating enantiomer amides in a racemic mixture of the formula:

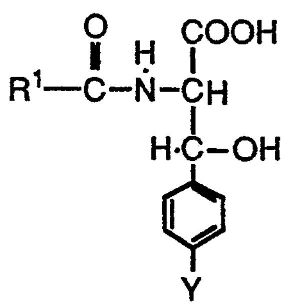

D-Threo
(S,R)

(V)

and

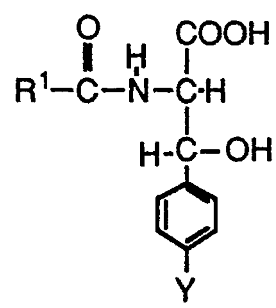

L-Threo
(R,S)

(II)

wherein $R^1$ is alkyl, substituted alkyl, alkenyl, substituted alkenyl, aryl, substituted aryl, aralkyl, substituted aralkyl, aromatic heterocyclic, substituted aromatic heterocyclic, heterocyclic alkyl or substituted heterocyclic alkyl and

Y is -H, $-NO_2$, $-S(O)xCH_3$ where x is 0, 1 or 2; comprising

(a) selectively hydrolyzing one of the enantiomer amides in the racemic mixture with $\gamma$-glutamyl hydrolase to its corresponding amine or salt having an enantiomeric purity of about 80 percent or greater while leaving the other enantiomer amide substantially unhydrolyzed; and

(b) separating the amine or salt from the substantially unhydrolyzed enantiomer amide.

2. The process of claim 1 wherein one of the enantiomer amides is hydrolyzed to an enantiomeric purity of

about 88 percent or greater.

3. The process of claim 1 wherein one of the enantiomeric amides remains unhydrolyzed to an enantiomeric purity of about 94 percent or greater.

4. The process of claim 1 wherein one of the enantiomeric amides remains unhydrolyzed to an enantiomeric purity of about 97 percent or greater.

5. A process of claim 1 wherein the enantiomer amide selectively hydrolyzed is the (S,R) enantiomer amide of formula (V).

6. A process of claim 5 wherein in step (b) the amine or salt is separated from the substantially unhydrolyzed amide by extracting the reaction mixture with a water immiscible organic solvent and separating the immiscible organic solvent containing the substantially unhydrolyzed amide from the reaction mixture.

7. The process of claim 6 wherein the pH of the reaction mixture being extracted is about 6 or lower.

8. The process of claim 5 wherein in step (b) the amine or salt is separated from the substantially unhydrolyzed amide via chromatographic processes.

9. The process of claim 5 wherein in racemic mixture (I), $R^1$ is phenyl and Y is $-S-CH_3$.

10. The process of claim 5 further comprising step (c), converting both the separated amine or salt and the substantially unhydrolyzed amide to a compound of the formula:

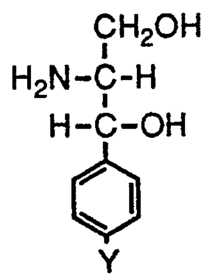

D-Threo
(R,R)

(III)

wherein Y is as defined in claim 1.